# EUROPEAN PATENT SPECIFICATION

(11) **EP 4 401 640 B1**
(45) Date of publication and mention of the grant of the patent: **09.12.2026**
(21) Application number: 22789178.5
(22) Date of filing: 13.09.2022
(51) Int. Cl.: A61B 8/08

(54) **VASCULAR IMAGING AND MEASUREMENT USING ULTRASOUND**
GEFÄSSBILDGEBUNG UND -MESSUNG MITTELS ULTRASCHALL
IMAGERIE VASCULAIRE ET MESURE À L'AIDE D'ULTRASONS

(30) Priority: 13.09.2021 US 202163243370 P
(43) Date of publication of application: 24.07.2024
(73) Proprietor: Foundry Innovation&Research 1, Ltd., Dublin 2 (IE)
(72) Inventor: WETTERLING, Friedrich, Dublin 2 (IE); SWEENEY, Fiachra, Dublin 2 (IE); BUXO, Teresa, Dublin 2 (IE); TUCKER, James, Dublin 2 (IE); GAUL, Robert, Dublin 2 (IE); MAGUIRE, Darragh, Dublin 2 (IE)
(74) Representative: Tomkins & Co
(86) International application number: PCT/EP2022/075458
(87) International publication number: WO 2023/037017

(56) References cited:
- WO-A1-2021/099449
- US-A1- 2013 303 915
- US-A1- 2020 211 174

## Description

### Field

The present disclosure is directed to imaging and measurement of the vascular system. In particular, it is directed to the measurement of the Inferior Vena Cava, IVC, from ultrasound imaging.

### Background to the Invention

Heart failure is one of the most significant chronic conditions afflicting adult populations. In the United States, 5.7 million Americans have heart failure, with 870,000 new cases annually. As the population ages, this population is growing, as approximately 10% of the population over 80 suffers from heart failure. It is estimated that by 2030, 8 million Americans will have heart failure. The cost of caring for heart failure is over thirty billion dollars per year. Twenty billion dollars of this cost is direct medical costs. This expense is expected to more than double over the next fifteen years.

In patients with chronic heart failure, a significant portion of these costs are due to hospitalization to manage acutely decompensated heart failure (ADHF). Each re-hospitalization can last up to a week, and costs approximately $10,000. ADHF is very often the result of some combination of a downturn in the heart's performance and excessive intake of fluids and/or salt. This leads to a build-up of fluid in the vascular system. Increased blood volume in the left atrium at higher pressure means higher blood pressure in the lungs, which eventually leads to fluid filling the lungs and an inability to breathe. At this stage it is imperative to hospitalize the patient to carefully manage them while drugs are delivered to remove the excess fluids.

Managing these patients to prevent the need for re-hospitalization is extremely challenging. Many non-invasive approaches to monitoring patients have been tried, such as weighing patients daily to detect fluid weight gain, having a nurse call them daily to assess their health status, and so on.

It is important to measure the onset of ADHF early enough to give the patient and/or caregiver enough time to adjust their behaviour, medication, or other factors to prevent the patient from ending up with acute congestion and the need for hospitalization.

Congestive heart failure is so named because additional blood volume backing up into the lungs causes fluid to seep out of the pulmonary circulation into the airway passages of the lungs, causing congestion of the lungs. The patients become short of breath, and typically need to be hospitalized and carefully managed while the excess fluid is removed by a combination of fluid management and aggressive use of diuretic medications.

Congestion occurs because the left ventricle is not able to pump all the volume of blood returning to the heart from the lungs. Although measurement of left atrial pressure, typically by measuring pulmonary artery wedge pressure, is commonly considered the most direct way to measure congestion in heart failure, there are other areas where congestion can be detected. When additional blood volume is added to the circulatory system, the inferior vena cava (IVC) is one of the first places for that added volume to collect. Lee et al, "Prognostic significance of dilated inferior vena cava in advanced decompensated heart failure" International Journal of Cardiovascular Imaging (2014) 30:1289-1295 states "In patients with advanced heart failure, left ventricular systolic dysfunction causes increased left atrial pressure. The pressure is transmitted back through the pulmonary circulation to cause pulmonary artery hypertension. The pulmonary artery hypertension can worsen pre-existing right ventricular dysfunction and exacerbate tricuspid valve regurgitation, leading to systemic venous congestion. If venous congestion and elevated central venous pressure are the hallmarks of heart failure, then distention of the inferior vena cava [by echocardiography] may be a good prognostic marker in patients with decompensated heart failure."

Heart Failure (HF) is a systemic and chronic disease which can involve many organs, including the liver, kidneys, and lungs. Continuous monitoring of the arterial and venous function, in addition to pulmonary function provides insight into the progression of the disease and its side effects.

Other volumetric related conditions and therapies such as dialysis, ultrafiltration, pulmonary hypertension, and hypertension may also benefit from such a system but for simplicity the detail below will solely focus on the HF application.

From venous perspective, diameter measurement of the IVC, proximal to the right atrium has been useful for identifying fluid status and this has been shown to be useful in the management of heart failure patients.

In a proposed novel application, the efficacy of this measurement can be further enhanced with the ability to discern the extent to which the congestion of the liver and/or kidneys contributes to the overall HF condition. Additionally, monitoring the structures and flowrates of the IVC, portal vein, renal veins and aorta provide additional detail on the function of the cardiovascular system and fluid status of a patient.

Haemodynamic congestion is a complex phenomenon and current high-end care involves the use of extensive, invasive measurements to estimate the total body water content and then estimate the proportions in each compartment (intravascular, extravascular, interstitial etc.).

From a venous perspective, diameter measurement of the IVC, proximal to the right atrium has been useful for identifying hemodynamic congestion and this has been shown to be useful in the management of heart failure patients.

In addition to correlation with right atrial pressure, the diameter of the IVC may correlate with renal function and renal sodium retention, which are also very important prognostic factors of heart failure. Therefore, increasing IVC volume and/or pressure may be a very effective early indicator of worsening heart failure condition.

Recent studies have indicated that the variation in IVC volume over the respiratory cycle is a more sensitive measurement of fluid overload and/or heart failure than simple measurement of average IVC volume, diameter, or pressure. During inspiration, intrathoracic pressure decreases, thereby increasing venous return and causing collapse of the IVC. During expiration, intrathoracic pressure increases, decreasing venous return and causing an increase in the volume of the IVC.

Since the IVC typically collapses in the anterior-posterior direction, some studies have suggested that the most accurate technique for measuring IVC volume changes with ultrasound is to measure the distance from the anterior wall of the IVC to the posterior wall of the IVC.

In applying this measurement to heart failure, at least one study has suggested that a variation of less than 20% (measured as maximum anterior-posterior dimension minus minimum A-P dimension, divided by the maximum A-P dimension) is indicative of ADHF.

While such vessel dimensions may be measurable using ultrasound, magnetic resonance imaging, computerized axial tomography, or other technologies, these imaging procedures must be administered in a hospital or other specialized facility, require expensive medical equipment, and skilled operators to obtain and interpret data and hence hamper daily monitoring in large populations.

As a result, the condition of a heart failure patient can worsen into a critical state before care providers become aware of it, dramatically increasing the mortality risk and cost of treatment for the patient.

If a heart failure patient can be monitored daily at home or in a remote location, this would enable the patient and physician to take proactive steps in time to prevent acute decompensation requiring re-hospitalization.

Magnetic resonance imaging and computerized axial tomography equipment is clearly limited to hospital use. Hospital ultrasound equipment is unsuitable for remote continuous monitoring due to its size, cost, and the need for trained operators, both for operation, correct transducer placement and image interpretation.

Use of a non-invasive, portable ultrasound device for vascular monitoring and intravascular volume management, which enables remote monitoring of vessel dimensions and flowrates without the requirement for a trained operator, is desirable. It is an aim of the invention to build a 3D model of the vascular system to identify structures and overcome the challenge of monitor positioning accuracy.

The multiple measurements obtainable with such a device would enable non-invasive estimation of the volumes of blood in the different areas. For example, the IVC diameter and collapsibility could provide an indication of vascular volume, IVC geometric response to other perturbations including manoeuvres such as breath hold, stand up from seated and others may provide information relating to the splanchnic activation or vascular tone as well as vascular volume, the portal vein diameter and flow rate could provide an indication of splanchnic activation and volume while the renal flow rates could provide an indication of renal function; all of these factors being useful in the diagnosis and treatment of vascular volume.

It is a further aim to improve the speed and accuracy of marking the outline of the inferior vena cava (IVC) and other structures in images acquired using external ultrasound (EXUS), intravascular ultrasound (IVUS), MRI and CT. Manual placement of markers on the outline of the vessel in the image is not only slow but also adds additional variability or error to the measurement.

The use of a module applied to the abdomen in the sub xiphoid position is described in WO 2013163605 A1. It is claimed that the module examines IVC collapsibility through the use of a cylindrical mechanical ultrasound array positioned on the patient by a medical professional in pre-hospital emergency situations. A collapsibility percentage is returned on the display of the device. The mechanical transducer may limit the imaged field of view leading to erroneous geometrical evaluation of the vessel, and, further, the need for placement of the device by a medical professional would limit its remote use, particularly in the home. The module would not provide any imaging data but just some descriptive data and is solely limited to IVC and Aorta measurements.

In addition to heart failure patients, haemodialysis patients have a chronic need for careful volume management. Large volumes of fluid are involved in the haemodialysis process and managing patients so that they don't end up hypovolemic or overloaded with fluid requires careful management. A monitor which provided immediate feedback on these patient's volume status before, during and after haemodialysis would be very helpful.

There are other groups of patients who might benefit from such a monitor. For example, patients in septic shock or occult shock due to trauma are subject to hypoperfusion which can be identified by measuring the degree of variation of the IVC with respiration (also referred to as collapse of the IVC), and fluid volume levels are key to the treatment of patients with Left Atrial Assist Devices (LVAD's). US2020211174A1 discloses quality indicators for collection of and automated measurement on ultrasound images. WO2021099449A1 discloses intelligent measurement assistance for ultrasound imaging and associated devices, systems, and methods.

### Summary of the Invention

The present invention provides a method of determining measurements of the Inferior Vena Cava, IVC, using ultrasound imaging according to claim 1.

The plurality of 2D ultrasound images may be obtained from a plurality of directions or locations on the body, by a body-worn ultrasound transducer array or other form of ultrasound transducer.

The plurality of 2D ultrasound images may be obtained using beam forming.

Identifying at least one anatomical landmark in the image may comprise the use of Doppler data to identify the direction and velocity of blood flow.

Identifying at least one anatomical structure in the image may comprise the use of known geometric properties of the at least one anatomic structure.

Performing image analysis on the 3D ultrasound image to identify the IVC relative to other anatomical structures may comprise identifying anatomical structures using edge detection.

The method may further comprise verifying the identification of the IVC in the image using an additional metric selected from a group comprising Doppler velocity, direction of blood flow, pulsatility, distances, pulmonary B-line measurements and audio respiratory data.

Determining the cross-sectional area of the IVC from the cross-sectional image of the IVC in the selected slice may comprise using edge detection techniques to identify the wall of the IVC.

Determining the cross-sectional area of the IVC from the cross-sectional image of the IVC in the selected slice may comprise the fitting of an ellipse to the IVC.

Determining the cross-sectional area of the IVC from the cross-sectional image of the IVC in the selected slice may comprise the use of thresholding techniques.

The method may further comprise the use of blob detection and analysis techniques to determine the cross-sectional area of the IVC from the cross-sectional image of the IVC in the selected slice.

The steps of providing, performing, selecting and determining may be repeated over time to obtain a series of measurements.

Determining the cross-sectional area of the IVC from the cross-sectional image of the IVC in the selected slice may comprise the use of a neural network to mark the IVC in the ultrasound image.

The invention further provides a computer program product comprising instructions which, when the program is executed by a computer, cause the computer to carry out the aforementioned method.

The invention further provides a computer-readable storage medium comprising instructions which, when executed by a computer, cause the computer to carry out the aforementioned method.

### Brief Description of the Drawings

Embodiments of the disclosure will be described, by way of example only, with reference to the accompanying drawings in which:
Figure 1a shows a method for vascular monitoring and intravascular volume management in accordance with one embodiment of the present disclosure.
Figure 1b shows a method for vascular monitoring and intravascular volume management in accordance with one embodiment of the present disclosure.
Figure 2 shows a comparison of marking done manually and automatically in accordance with one aspect of the present disclosure.
Figure 3 shows a comparison of area traces, point clouds and histograms between manual and automated marking of the data from figure 2 in accordance with one aspect of the present disclosure.
Figure 4 shows one embodiment of a semi-automatic marking process in accordance with the present disclosure.
Figure 5 shows a further embodiment of a semi-automatic marking process in accordance with the present disclosure.

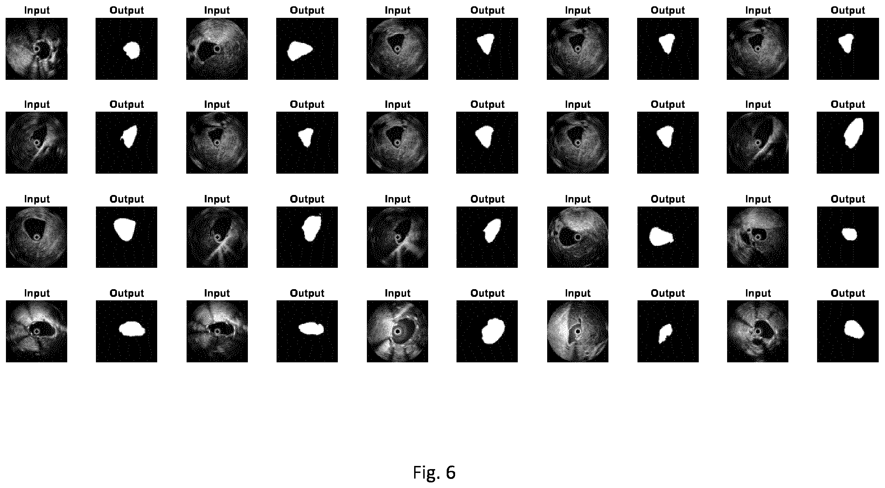

Figure 6 shows some sample outputs from a neural network trained algorithm embodiment.
Figure 7 shows a 3D representation of a vessel reconstructed from stacked, marked IVUS images.
Figure 8 shows an edge extraction technique in accordance with one embodiment of the present disclosure.
Figure 9 demonstrates a method of training a neural network in accordance with one embodiment of the present disclosure.
Figure 10 demonstrates shows an example graph showing improvements with training iterations in accordance with one embodiment of the present disclosure.
Figure 11 shows an image showing marked location in accordance with one embodiment of the present disclosure.
Figure 12 is a screenshot of GUI showing display of ultrasound with computed edge located by a machine in accordance with one embodiment of the present disclosure.
Figure 13 shows an ultrasound image sectioned into smaller sized images as part of an algorithm in accordance with one embodiment of the present disclosure.
Figure 14 shows a binarized section of the ultrasound image of figure 13 in accordance with one embodiment of the present disclosure.
Figure 15 shows binary maps of the strut location from the ultrasound image of figure 13 in accordance with one embodiment of the present disclosure.
Figure 16 shows edge extraction results from the binary maps of Figure 15 in accordance with one embodiment of the present disclosure.
Figure 17 shows the true location of the struts assigned to the ultrasound image of figure 13 in accordance with one embodiment of the present disclosure.
Figure 18 shows an ellipse fitted to the red dots of the image shown in Figure 17 in accordance with one embodiment of the present disclosure.
Figure 19 is a flow diagram of an algorithm in accordance with one embodiment of the present disclosure.
Figure 20 is an example trace output for area as a function of time in accordance with one embodiment of the present disclosure.
Figure 21 shows a portable ultrasound device in accordance with one embodiment of the disclosure.
Figure 22 shows the ultrasound transducer of the portable ultrasound device of Figure 21 in greater detail.
Figure 23 shows a cross-sectional view of transducer securement means in accordance with one embodiment of the disclosure.
Figure 24 shows a cross-sectional view of transducer securement means in accordance with an alternative embodiment of the disclosure.
Figure 25 is a front view of the ultrasound transducer securement means of Figure 24 attached to a patient's torso.

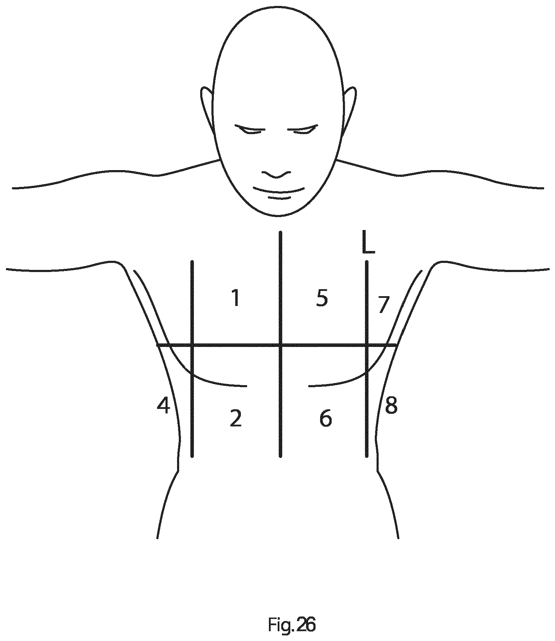

Figure 26 shows suitable locations for B-line monitoring.

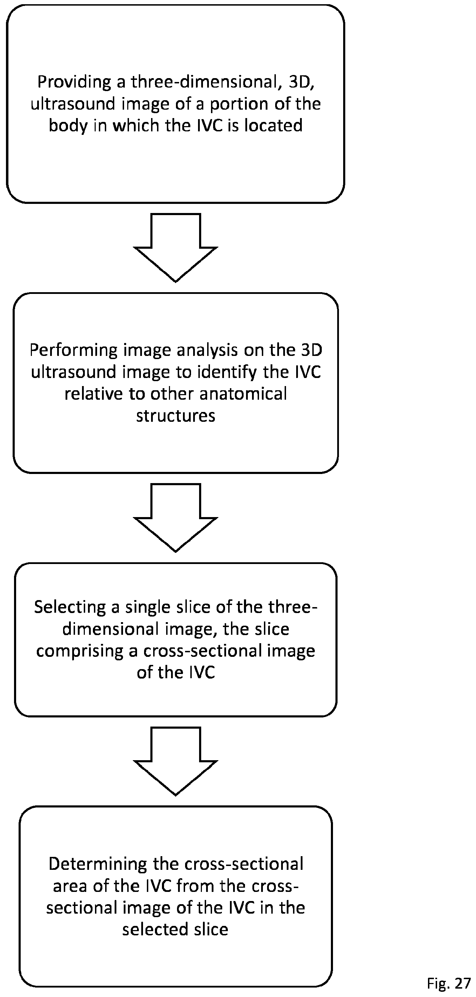

Figure 27 shows a method of determining measurements of the Inferior Vena Cava, IVC, using ultrasound imaging in accordance with the present disclosure.

### Detailed Description of the Drawings

The present disclosure uses ultrasound imaging in combination with advanced image processing to identify the appropriate structures, determine measurements. Clinical status may be inferred from these measurements, to enable remote diagnoses and the information may be reported to a clinical team to aid in the monitoring of HF patients.

The techniques/processes described herein provide a non-invasive, automated evaluation of the volumetric flow dynamics and geometry of the abdominal vasculature, both arterial and venous, from the level of the Aortic and IVC bifurcations cranially to the right atrium of the heart. Further, cardiac output and pulmonary function may also be monitored.

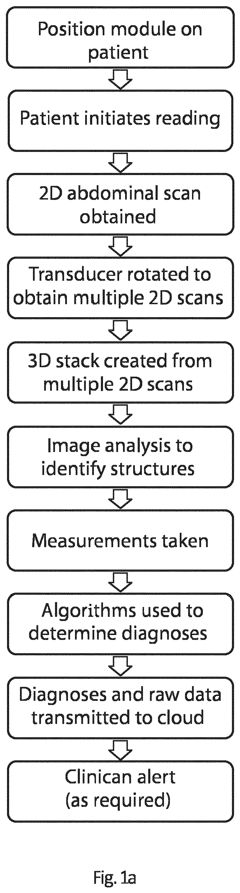

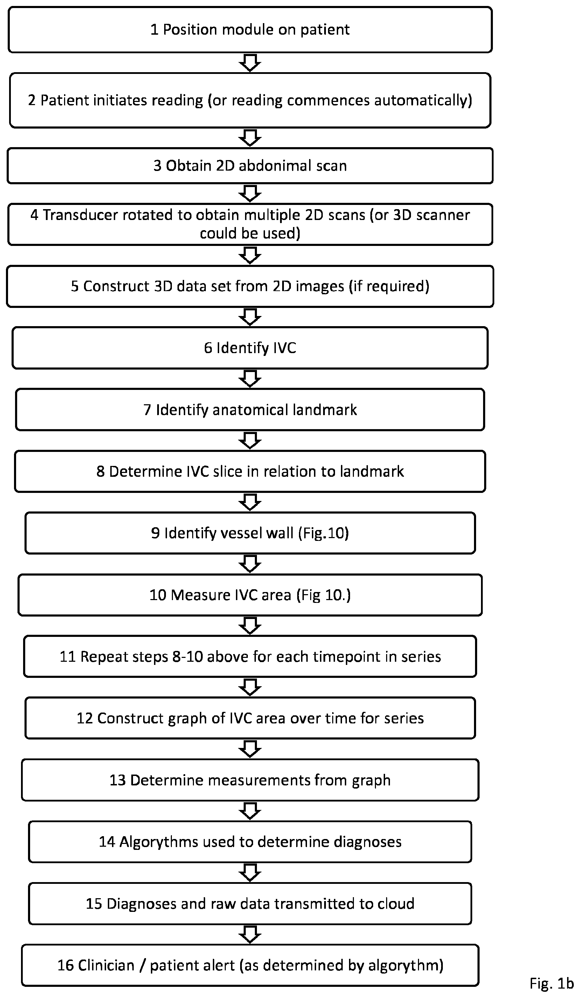

Figure 1a describes an overview process by which ultrasound images can be processed to identify and extract useful IVC metrics to inform treatment of patient for fluid related conditions in accordance with one embodiment of the disclosure. Figure 1b describes a more detailed process by which ultrasound images can be processed to identify and extract useful IVC metrics to inform treatment of patient for fluid related conditions in accordance with one embodiment of the disclosure. Step 1 describes the positioning of the module on the patient. The patient is preferably lying supine before initiating a reading by pressing the activation button. The accelerometer element is used to confirm patient position. Step 2 describes the initiation of the reading. This could be commenced automatically based on a time or accelerometer input within the module or may be initiated by the patient. Audio or other cues can be given to indicate the stage of the process or initiate breathing techniques by the patient. Step 3 describes the process for obtaining multiple 2D abdominal scans. The ultrasound transducer scans the abdomen and obtains at least one 2D abdominal scan. In step 4, the transducer aperture is then rotated or tilted to obtain at least one additional 2D scan in a different plane to the first. This angulation could also be achieved using beam forming. In step 5, 3D stacks are then created from the multiple 2D scans.

Step 6 describes the use of image analysis to identify structures such as the aorta, the IVC, renal arteries, veins including hepatic veins, the right atrium, and the diaphragm. A number of factors could potentially be used to identify the relevant structures, such as Doppler to identify the direction and velocity of blood flow with the IVC flow being cranial and the aorta flow being caudal. In another embodiment, the movement and/or thickness of the vessel walls could be used for differentiation, with the IVC having a more mobile, thinner wall, while the aorta is a thicker less mobile wall. Another differentiator may include the temporal dynamics of the flow or vessel wall signals, with the IVC containing strong respiratory and cardiac elements while the aorta has a more predominant cardiac influence. In step 7, one or more anatomical landmarks are identified in the 3D image stack. Anatomical landmarks may identified using multiple techniques including, but not limited to edge detection and/or Doppler in the identification of branches. In step 8, a single ultrasound slice containing the IVC, located a known distance from the previous identified landmark/s, is chosen. Having identified a structure, or a combination of them, the device can be used to make additional measurements. Additional measurements might include Doppler velocity, direction of blood flow, pulsatility, distances, pulmonary B-line measurements and audio respiratory data.

It will be appreciated that such additional metrics may be used in the overall/final assessment of the vessel. For example, blood velocity from Doppler could be incorporated into an alert in combination with IVC dimension data. Blood velocity profile (speed and direction) over the respiratory and cardiac cycles could be informative in relation to fluid status. For example retrograde flow in the IVC may be indicative of valvular regurgitation while flow dynamics may provide information on congestion. Velocity can be measured if Doppler ultrasound is deployed. The knowledge of IVC segment in the brightness ultrasound image allows for extracting velocity profiles for IVC in conjunction with measures of Doppler ultrasound information.

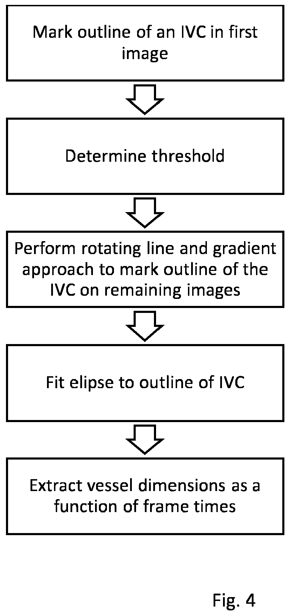

In step 9, the single slice chosen is analysed to identify the IVC vessel wall using techniques including but not limited to edge detection and gradient analysis. Step 10 determines the IVC cross sectional area. In one embodiment, this may be determined by calculating the cross-sectional area of an ellipse fitted to the image of the IVC as defined in Figure 4. In another embodiment, thresholding of the identified vessel coupled with blob detection and analysis may be used to determine the IVC cross-sectional area. In step 11, steps 8-10 are repeated for each time point in the time series. Where multiple frames are recorded for a given time point, slices located at the same known distance from landmark(s) are used. In step 12 a graph of IVC cross-sectional area is plotted over the time series recorded. Step 13 describes how key features such as area minimum, mean, maximum and collapsibility can be measured from the time area time series. Step 14 describes how an algorithm may use these features to diagnosis a patient. In one embodiment, this diagnosis may be based on individual features breaking a feature threshold. In another embodiment, this diagnosis may be based on a feature trending in a particular direction or fluctuating to a greater than allowable tolerance over a period of time. In step 15, this diagnosis is as well as the raw and processed data is transmitted to the cloud allowing for remote access by healthcare professionals (HCP). Step 16 describes how the clinician or HCP would be alerted if required, as determined by the algorithm.

The following are examples of additional metrics which may be recorded:
- Volumetric flow rate of the aorta or IVC using ultrasound (pulse or continuous wave or Doppler) optical (visible or infra-red) or magnetic resonance imaging (MRI);
- Blood flow pulsatility, using ultrasound (pulse or continuous wave or Doppler), optical (visible or infra-red) or MRI;
- Uniformity of the aorta over its length, in order to monitor for any Abdominal Aortic Aneurysms (AAA) using ultrasound, X-ray, Computer Aided Tomography (CT) or MRI. An aneurysm is usually defined as an outer aortic diameter over 3 cm (normal diameter of the aorta is around 2 cm), or more than 50% of normal diameter. If the outer diameter exceeds 5.5 cm, the aneurysm is considered to be large;
- Volumetric flow rate of the aorta over the length of what is visible, using a combination of sensing modalities listed above;
- Pulse pressure for evaluation of aorta stiffness;
- Maximum flow rate of the aorta (using CW Doppler) and Abdominal Aortic Waveform, indication of the cardiac output;
- Volumetric flow rate of the renal artery;
- IVC to Aorta ratio;
- IVC collapsibility near the right atrium (volumetric) with cardiac or respiratory cycles. A measurement of volume collapsibility could be returned, in addition to the conventional measurement for collapsibility (Vol/Diameter: Max-Min/Max). The absolute values of the diameter and cross-sectional area of the IVC could be trended;
- Respiratory rate analysis using the ultrasound signals, accelerometers / gyroscopes in the ultrasound transducer;
- Cheyne-Stokes respiration using the sensors listed above;
- Volumetric flow rate of hepatics;
- Volumetric flow rate distal to the hepatics;
- Volumetric flow rate of the renal veins;
- Volumetric flow rate distal to the renal veins;
- B-lines in the lungs using ultrasound;
- A volume collapsibility measure of the IVC between the RA and hepatics, at 1cm intervals. Further, the length and longitudinal strain of the IVC, along its imaged length could be recorded, between the RA to hepatics, hepatics to Renal, etc.
- Subxiphoid view of the heart to evaluate cardiac output;
- Continuous monitoring of cardiac output using a combination of the sensing modalities listed above.

As listed above in respect of step 6, the pulsatility of identified structures or direction of flow may be used for example to identify the Aorta and IVC to be blood vessels. A Doppler velocity evaluation would show there to be blood flow in these two large vessels, with the Aorta identified as the vessel having a higher maximum blood flow velocity. The direction of blood flow in the vessels would also serve to confirm the identity of the vessels, the Aorta having a blood flow direction from the heart in an opposite direction to the IVC. The technique may also evaluate the shape of the vessels, the Aorta appearing circular and the IVC more ellipsoidal. This detail would be recorded.

Having identified the IVC and Aorta in the initial transverse image, the same identification process is carried out on further 2D B-mode images acquired in the same plane and on images acquired above and below the initial 2D plane, to serve as confirmation of the IVC and Aorta locations. Once the IVC and Aorta are identified, the 3D transducer array begins to map the IVC and Aorta and surrounding area using b-mode ultrasound. Mapping is conducted for different times in the respiratory cycle. The anatomical map could be used as a basis for estimating the volume of the vessels.

Multiple 3-d volume and Doppler maps are acquired over multiple respiratory and cardiac cycles.

Pulsed Wave or Colour Doppler ultrasound could subsequently be used to estimate the blood flow velocity at the centre of the identified vessels (identified as the maximum flow rate) and at the edges of the vessel walls (as the minimum flow rate), from which a mean flow rate is recorded. Combining the volume and the blood flow velocity, the volumetric flowrate at each point along the IVC and Aorta could be estimated.

The system could prompt the patient to inhale and exhale with the respiratory cycles automatically identified from the ultrasound images, through examination of the IVC length between the hepatic veins and the right atrium. With inspiration the increased intrathoracic cavity pressure causes a flattening of the IVC, therefore it becomes possible to use the ultrasound device to determine collapsibility of the IVC.

Once the IVC and Aorta have been identified from the 3D image it is possible to identify veins and arteries leading into Aorta and IVC. For example, the ultrasound response of a kidney being at one end of a vein can identify the vein as a renal vein. The flow direction and expected distances from other vessels could also be used to confirm identifications. In another example, identification of the right atrium and then the diaphragm can be used to confirm that the next lumens below that entering the IVC are therefore the hepatic veins.

Ideally the length of the IVC imaged includes branching of the hepatic and renal arteries from the liver and kidneys, respectively. Therefore, evaluating the blood flow velocity profile and vessel volume profile, combined, provides a measure of the volumetric flow rate along the IVC. This helps to characterise the level of fluid responsiveness and to indicate the congestion contribution from each of the liver and kidneys.

Algorithms are then used to determine diagnoses. For example, the processor could compare results to previous results and make therapeutic decisions/ recommendations based on pre-programmed criteria and limits. Automated algorithms are used to determine specific diagnoses. These may be adapted from existing guidelines (ref IVC collapsibility in echo guidelines) or may solely be enabled based on the present disclosure.

The algorithms undergo an initial machine learning/training phase in order for reproducibility to be ensured. In this training phase, the placement of the transducer is carried out on multiple occasions, establishing a range of depths/shapes/flow rates of the landmark structures.

Upon the completion of the diagnosis portion the data is then communicated by the communications interface from the module to the cloud via GSM, wireless, Bluetooth^{®} or similar. The raw data and diagnosis data are both transferred. In another embodiment a subset of the data is transferred in order to minimize the communication burden.

In one embodiment, the data is stored in the cloud and is accessible by nursing or clinical staff via a web portal. Alerts are pre-programmed based on specific thresholds being exceeded and physicians are alerted of a change in patient status, thus facilitating a modification in therapy.

Other volumetric related conditions and therapies such as dialysis, ultrafiltration, pulmonary hypertension, and hypertension may also benefit from the portable ultrasound device of the present invention. In the dialysis and ultrafiltration applications, the ultrasound module could be linked to the dialysis filtration machine thus providing a feedback loop to monitor the physiological variables and modulate the parameters on the machine to optimize the dialysis process.

In the hypertension examples the device could be used to predict elevated pressures in the patient via metrics such as the IVC collapsibility and this information communicated to the clinical team to inform the treatment of the patient.

Another potential use for the present disclosure is to monitor the progression of aortic aneurysms. These weakened, enlarged sections of the aorta can be potentially deadly if they rupture. This device would facilitate the daily monitoring of any changes / progression / growth / dilation of the aneurysm remotely.

In accordance with the present invention, it is also possible to discern the extent to which the congestion of the liver and/or kidneys contributes to the overall HF condition. Additionally, monitoring the structures and flowrates of the IVC, portal vein, renal veins and aorta provide additional detail on the function of the cardiovascular system and fluid status of a patient. Further, information on the respiratory rate, its variability and pattern can prove useful in identifying progression and specific type of HF. Continuous monitoring of the arterial and venous function, in addition to pulmonary function, provides insight into the progression of the disease and its side effects.

### Ultrasound Image Extraction Algorithm

The present disclosure further provides systems and methods for marking ultrasound images to detect the IVC and other structures of the vascular system. Manual placement of markers on the outline of the vessel in the image is not only slow but is also adding additional variability to the measurement. The present disclosure utilizes an automatic algorithm to mark the vessel while supervision is possible in order to intervene if the fit result is insufficient. In the semi-automated embodiment, the first image of every sequence is marked manually, and corrective action entails manually marking the vessel at rare occasions. In the fully automated version user input is not required.

For each function (lumen IVUS, strut IVUS, and lumen EXUS) three data sets were chosen as test datasets for which manually marked trace data of the cross-sectional vessel area and required marking time was available.

The validation result showed that the new marker technique marks 3-6 images per second while manually marking requires at least 10 seconds per image. The difference of the means between manual and semi-automatically marked images was 14mm2, the standard deviation from the mean was 6mm2 for the semi-automatic marker while it was 115mm2 for the manual marking approach.

Images of the cross-section of the inferior vena cava (IVC) acquired using external ultrasound (EXUS) and intravascular ultrasound (IVUS) are a useful resource to know the native behaviour of the IVC and check that the area measurements taken by the FIRE1 sensor are accurate enough. However, marking the outline of the inferior vena cava (IVC) in these images could be a long and tedious task if done manually. Moreover, the noise level in this type of image is higher than in others, which makes that totally automatic marking potentially introduces a lot of errors.

A Graphical User Interface (GUI) was developed to enable a semi-automatic marking which allows performing automatic marking with real-time correction possibility in case of errors in the marking. Three algorithms are included in this GUI which enables the marking of the struts of the sensor in IVUS (Struts Marker), the marking of the IVC lumen in the IVUS (Lumen Marker) and the marking of the IVC lumen in external US images (External US marker). The advantages of this tool can include accuracy, speed, and more human independence, among others; which facilitate the work of the operator.

We hypothesize that the semi-automatic marking has also lower probability of introducing errors than the other type of markers (Table 1). It will be demonstrated/verified herein that this tool is not only better in terms of usability for the user, but also in terms of accuracy.

**Table 1. Sources of error introduction for each type of marking. Scaled as low, medium, and high probability of these error to occur.**

| | Manual Marking | Automatic Marking | Semi-Automatic marking |
|---|---|---|---|
| Bad detection of IVC | Low | High | Low |
| Operator fatigue | High | Low | Medium |
| Incorrect labelling | Medium | Medium | Low |
| User unexperienced | High | High | Low |

### Data

Three datasets of US/IVUS images for each algorithm of the GUI were selected as examples of small, medium, and large IVC area (9 datasets in total). IVUS images were extracted from pre-clinical experiments in sheep, whereas external ultrasound was sourced from anonymized human data. All participants provided written informed consent to use their data for further analysis.

### Marking procedure

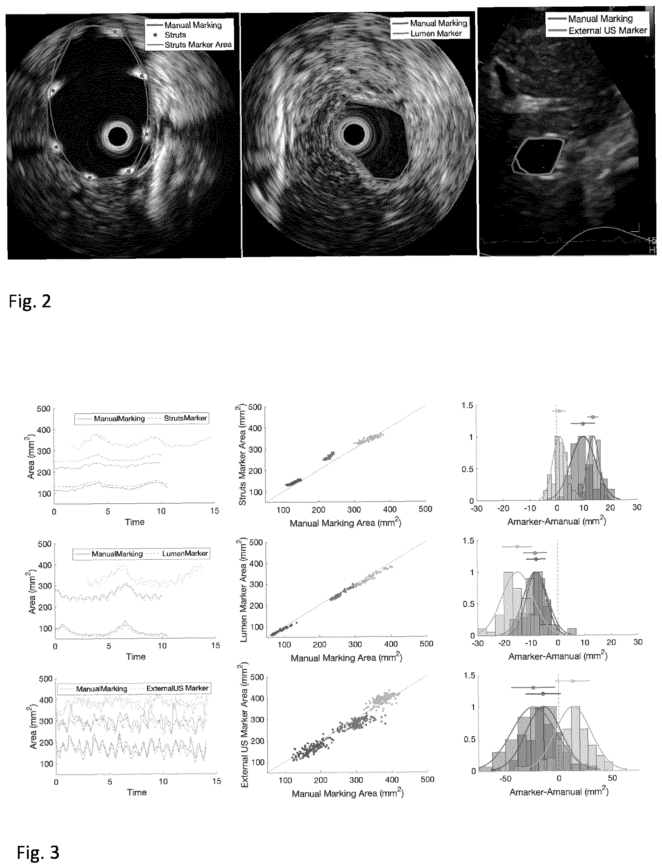

The marking concepts described herein can be applied to a number of different ultrasound imaging techniques, including IVUS and EXUS. IVUS imaging can produce cross-sectional views of the vessel as per Figure 2. Figure 2 shows an example of marking done manually and with the GUI markers.

EXUS imaging can also produce similar cross-sectional views, as well as longitudinal sections of the vessel. The techniques described below can be applied to IVUS and cross sectional EXUS images.

Manual marking and Semi-Automatic marking with the GUI of the cross-sectional vessel area were performed in all these datasets as shown in Figure 4. The main characteristics from these markings are summarized in Table 2.

Manual Marking was done in 1 every 5 images for all the algorithms, which means that some temporal resolution is lost. The operator identifies the IVC at a glance and marks it with points. The IVC area extracted from the images is the area of the oval defined by the marked points.

Semi-Automatic marking was performed for all the images by means of the developed GUI. The operator marks the first image, and the algorithm automatically identifies the subsequent images. In case of mismarking, the operator can always pause the algorithm, rectify the marking, and continue automatically.

**Table 2. Main characteristics of each type of marking.**

| | Manual Marking | Struts Marker | Lumen Marker | External US marker |
|---|---|---|---|---|
| Temporal resolution | ~6 images/s | ~30 images/s (IVUS resol.) | ~30 images/s (IVUS resol.) | ~46 images/s (US resol.) |
| Speed | ~10 images/min | ~150-180 images/min | ~250-300 images/min | ~250-300 images/min |
| Human dependent | All time | Intermittent | Intermittent | Intermittent |

Figure 4 shows a flow diagram representing an edge detection algorithm for semi-automatic marking in accordance with one embodiment of the disclosure, based on images acquired using external ultrasound (EXUS) and/or intravascular ultrasound (IVUS).

The process starts by operator marking of the outline of the IVC in a first image of a set of images. The operator would be presented with the first image in a time series and be prompted to mark the outline of the IVC in the image. The semi-automated system would then determine the threshold to find edge matching marked edge using a rotating line and gradient approach. The system uses the location of eight marked locations that must be placed on the edge of the IVC in the image. The centre point of those locations will be determined by fitting an ellipse to the coordinates. Then a line is drawn through the center point and each marked position. The gradient of the pixel values along the line is computed and the edge is determined as the location at which the gradient is the largest. The threshold for the gradient is then determined as a percentage of the found value in order to locate the edge in the subsequent images. The system would then proceed to perform sequential rotating line and gradient analysis of the image to mark the outline of the remaining images. This involves the system taking a line from outside the marked IVC wall to the centre of the IVC. The greyscale values of the image along this line are evaluated and the point at which the largest change in these values is identified. This colour change identifies the vessel wall, and the system records the location of this point. The line is then rotated a number of degrees and the process repeated to identify the next point of maximum colour gradient and this point is recorded as another point on the vessel wall. This process is repeated in angular increments up to 360 degrees and the points identified are joined to define the IVC wall.

The outline of the IVC is then marked automatically on the consecutive and remaining images. An operator can observe these results and intervene in the case of any mismarking. The outline may be remarked to improve accuracy.

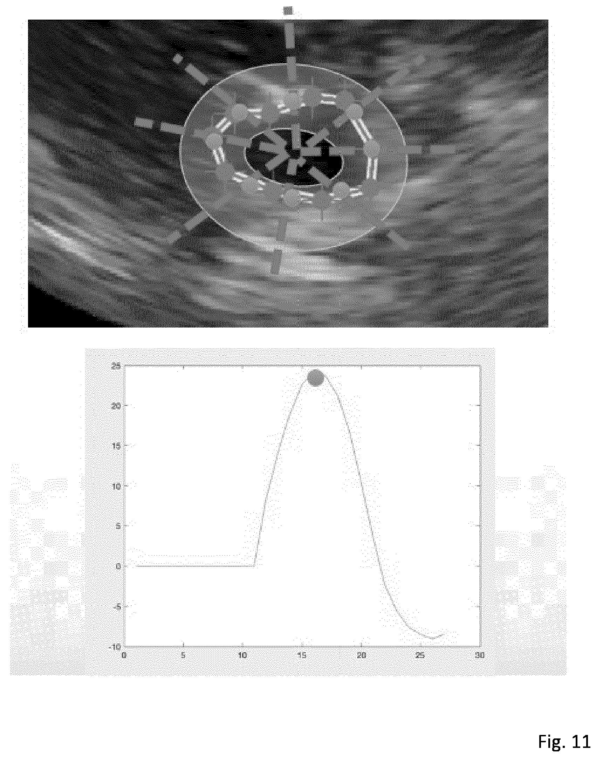

Figure 11 shows an image showing marked location (purple). The center point (intersection of all green lines) is found after fitting an ellipse to the purple points. The ellipse is then divided in subsections of equal opening angle. The gradient is computed along the line (bottom graph) and the edge is determined as the maximum gradient value along each line. The threshold for each line is stored as a percentage of the found gradient value to determine the edge location in subsequent images.

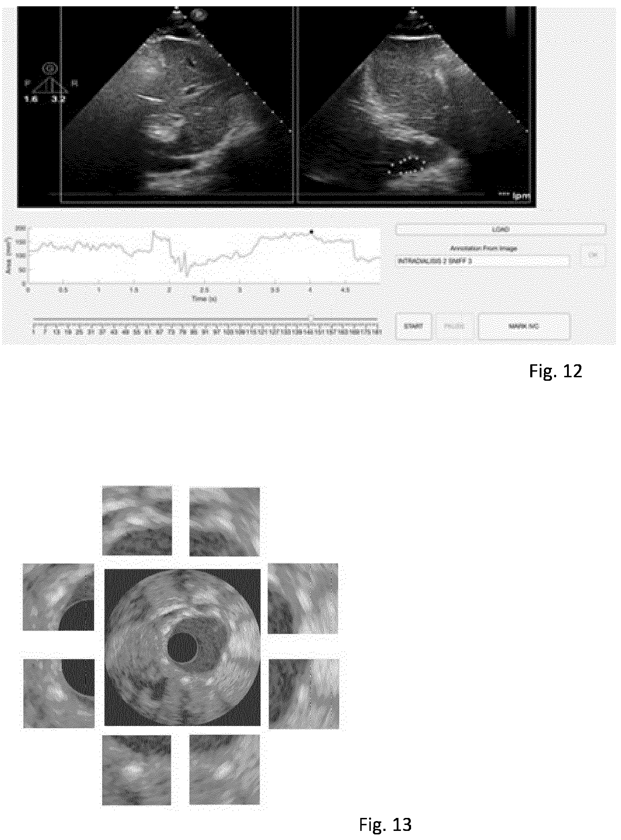

Figure 12 is a screenshot of GUI showing display of ultrasound with computed edge located by a machine for the observer/operator to assess and to intervene in case of mis-localisations.

When the data set is complete an ellipse is fitted to the outline of the IVC. The shape is transformed into a set of Cartesian coordinates with x- and y-position. An ellipse is fitted using a minimum norm approach.

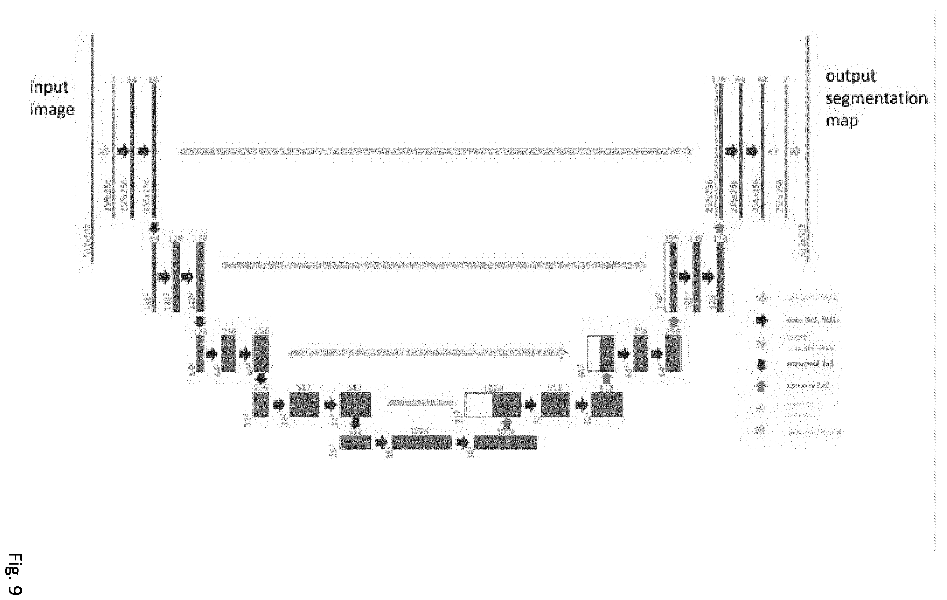

Finally, vessel dimensions such as major and minor axis, and area are extracted from the resulting images. The area and the diameters of the minor and major axis of the IVC, plus the area of the outlined region may be extracted. As the frame rate of the ultrasound is defined and constant, this can be used to plot the area and diameters over time. These results can then be presented in a graphical representation over time such as shown in figure 9. Top row: Struts Marker, Middle row: Lumen Marker, Lower row: External US Marker.

The semi-automated nature of this system relates to the partial involvement of the user in the marking process. This user involvement can be in the marking of the initial IVC image or in other embodiments could be in the marking of points inside and outside the vessel in initial images, it could also involve the reviewing of images as they are marked by the system as a quality control step.

An automated embodiment is also described where the marking process is fully automated. In this instance the centre of the image is automatically detected as it is the location of the IVUS transducer (@half width of image and half height of image, using an assumed diameter of 3mm of the catheter plus artefact in conjunction with the information of the dataset on pixel size) and the marking process is completed without the intervention of the user. It is envisaged that additional quality check process steps in the system could be added to increase the accuracy of the automated version.

The IVUS marking systems described above could also be used in case where an implant or sensor is placed within the vessel (Fig 2). In this instance the systems would involve the marking of the struts of the implant or sensor.

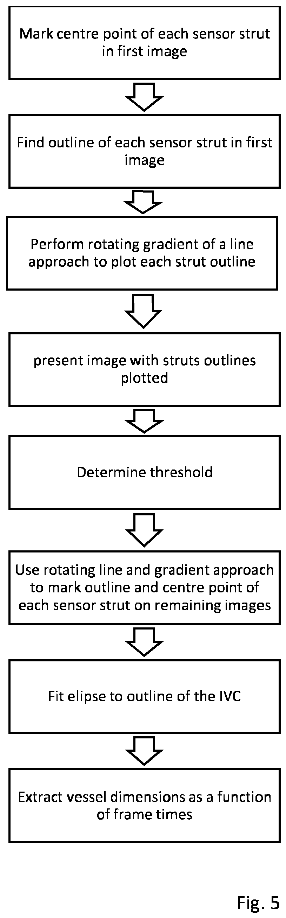

Figure 5 shows a flow diagram representing an edge detection algorithm for semi-automatic marking in accordance with another embodiment of the disclosure, based on images acquired using intravascular ultrasound (IVUS) with an intravascular sensor in situ. The process starts by loading a first image and the operator prompted to mark the centre point of all visible struts. In the semi-automated version of this, the user could mark the white strut regions in the first image and the system use these starting points to identify the area of maximum white, close to the starting point locations in each subsequent image. It could also use the identified strut location in each image as the starting point for the search for maximum white in each subsequent image.

The algorithm then automatically finds the outline of the struts and presents to the operator an image with all strut outlines plotted. This is performed by using a rotating gradient of a line approach. A threshold is then determined for each strut to be used in consecutive images. The algorithm finds the outline of struts and respective centre points automatically in subsequent images of the set of images. By using the centre points of the struts, the lines are spread out equally, therefore any individual marking error is reduced. In other embodiments the marked locations can be unevenly spread. An operator can intervene in the process if any of the strut locations are mislocalised, in which case the operator can manually update the centre strut locations.

When all of the strut locations have been marked, again an oval could be fit to the shape and dimensions extracted and a trace over time outputted. From the strut locations the algorithm fits an ellipse and returns area, minor and major axis diameters as a function of respective frame times. In one embodiment, to estimate the true outer area of the sensor and considering that the counterpoints of the struts are localises, 0.5mm is added to the minor and major diameters at both ends to compute an area that is most reflective of the outer sensor volume as this represents the distance between the centre of the struts and the vessel wall.

The struts' location is known from the first image. Only a sub-section close to the marked location is extracted (~.5cm by .5cm). To find the pixel value threshold to be used for each location the threshold is varied from 0.4 to 0.9 in 0.1 steps in images with pixel values scaled between 0 and 1. The images are binarized using the various possible thresholds. An edge detection filter is used to find the number of pixels in the vicinity of the marked strut location (within 1mm). The threshold is found by finding the region with ~1mm diameter in 1mm distance to the marked strut location. This threshold is variable for each strut. Once again, this system could be made automatic in a similar way to that described above.

As shown in figure 13, an image is sectioned into smaller sized images using the information (location) of marked struts.

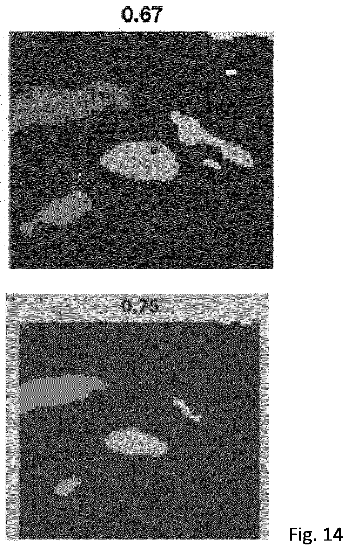

With reference to figure 14, the threshold is varied stepwise to binarize the subsection. The algorithm checks the nearest closed surface to the marked location (green) and records the surface area. The threshold is found for the area that is the closest to an area with ~1mm2 area and a center of mass no further away from the marked location than 1mm.

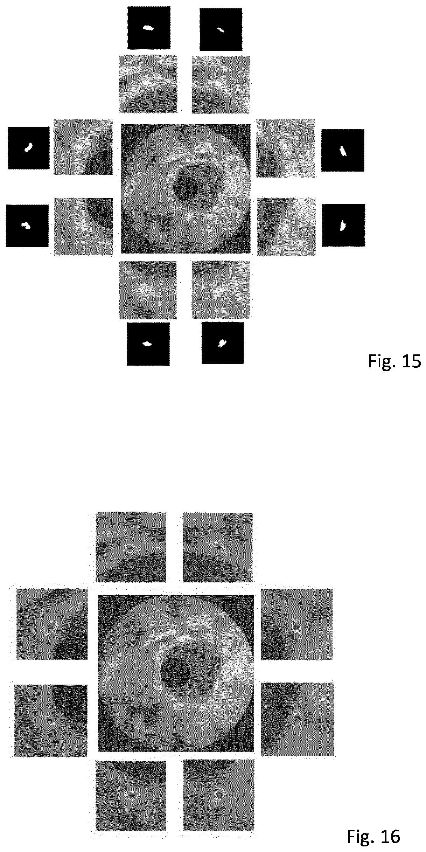

This process is repeated for each subsection. Resulting in binary maps of the strut location (black and white maps) as shown in Figure 14. As shown in Figure 15, the edge is extracted from the binary maps and the center of mass is determined.

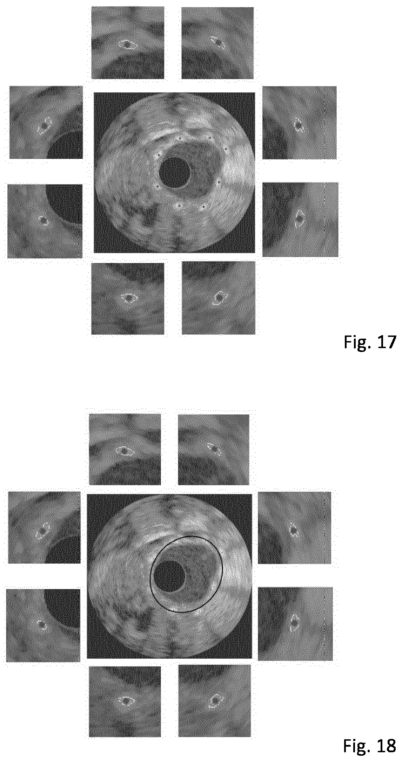

As shown in Figure 17, the found center of mass is the true strut location and is assigned to the image (red dots). An ellipse is fitted to the red dots and area is derived from this fit as well as minor and major axis diameters as shown in Figure 18.

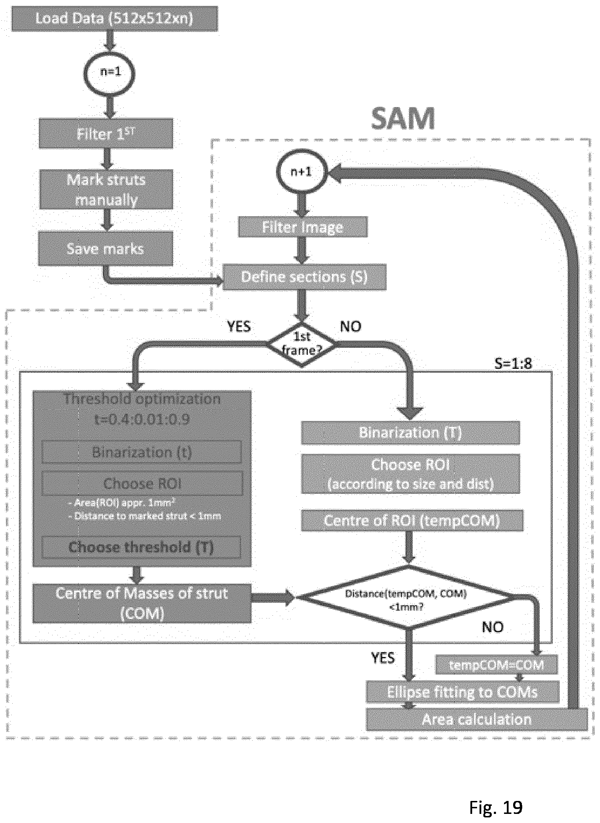

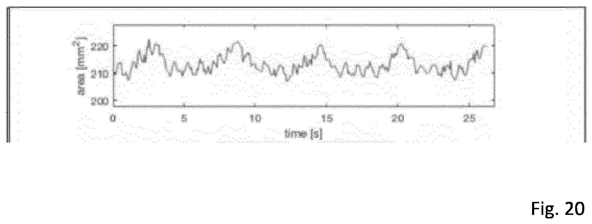

The algorithm is explained in the flow diagram of Figure 19. An example trace output for area as a function of time is shown in Figure 20.

In other embodiments a similar gradient-based approach could be used to mark longitudinal EXUS images and extract time series data on the measured diameter of a vessel.

The marking process of the present invention may result in the generation of an IVC trace depicting the motion of the IVC overtime (as shown in Figure 3) These traces communicate relevant information such as the IVC mean area and IVC respiratory collapse easily.

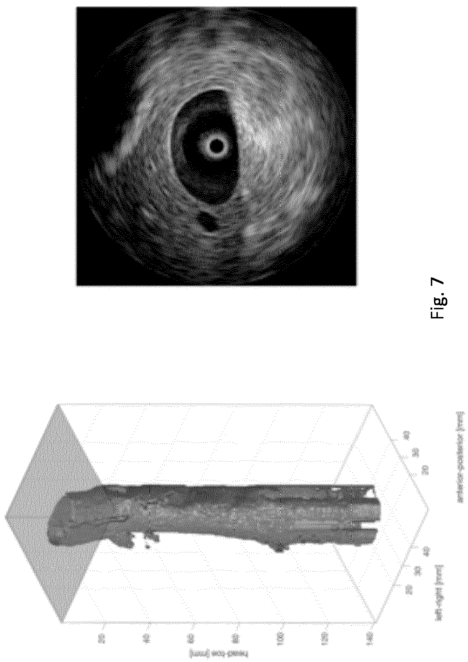

Where an intravascular ultrasound system is used, a pullback reconstruction of the IVC is possible as the intravascular ultrasound system is withdrawn (pulled-back) at a constant speed through the IVC (as shown in Figure 7). The images are then marked using the systems previously described and the resulting individual images can then be stacked to provide a 3D image of the vessel.

In accordance with some embodiments, neural networks are used in the marking process to identify the IVC.

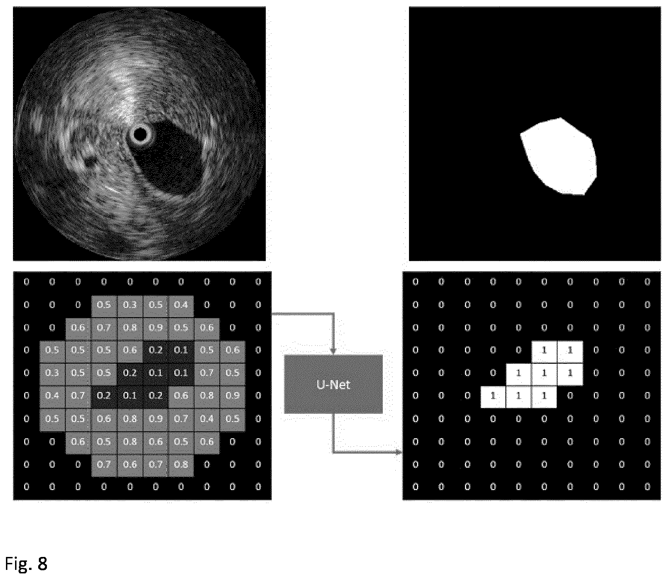

With reference to Figure 8, the objective is to extract the edge of the image provide in the left top corner. The right top corner image shows a manually marked vessel (white). Below images show how this is achieved conceptionally binarizing the grey value image using a trained neural network (U-Net).

As shown in figure 9, images with labels are used to train the network. Operations in the network are decided before learning (e.g., convolutions). Variables (weights) used in the operations are initially randomised. Error in prediction is calculated and this is used improve the variables.

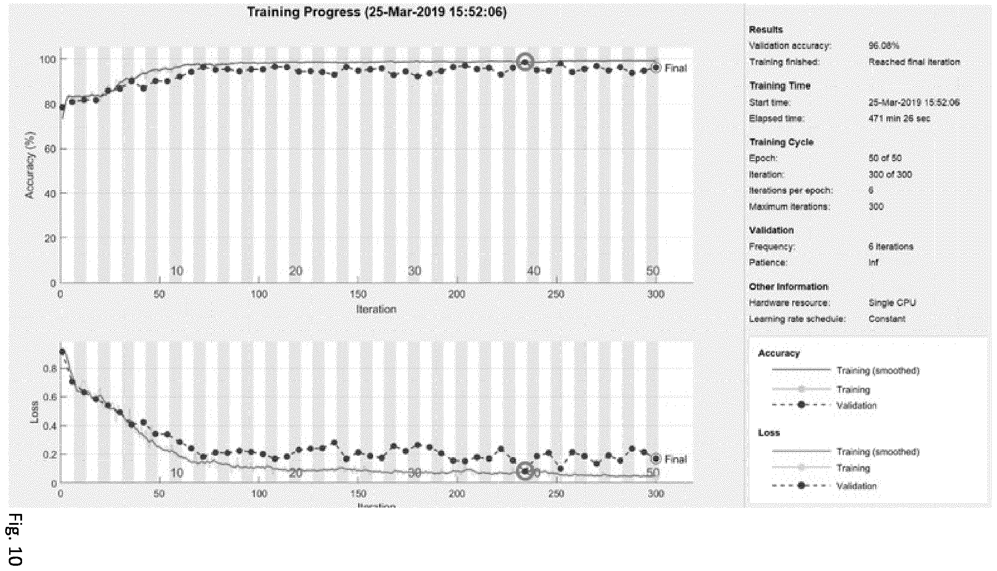

Figure 10 shows an example graph showing improvements with training iterations until optimised set of model parameters is determined.

### Accuracy testing

To validate the tool, the difference between the area extracted from manual and the markers was computed for all the data points. Mean and standard deviation were extracted and compared to the ideal results, which should be as close to 0 as possible. These measurements are used to ensure that the markings and calculations performed by the algorithms in the GUI are reliable and accurate.

### Results

An area trace was extracted from each of the three datasets of small, medium, and large area from each of the manual and GUI marked data sets and compared (Figure 2). The characteristics of each of the traces are summarized in Table 3.

**Table 3. Manual and markers traces characteristics.**

| | | Manually Marked (mm²) | GUI Markers (mm²) |
|---|---|---|---|
| Struts Marker | Small | 128.30 ± 11.68 | 140.76 ± 8.65 |
| | Medium | 229.43 ± 8.45 | 261.15 ± 10.61 |
| | Big | 336.38 ± 19.56 | 339.99 ± 13.54 |
| Lumen Marker | Small | 82.40 ± 17.90 | 74.19 ± 15.02 |
| | Medium | 261.08 ± 22.14 | 252.83 ± 22.77 |
| | Big | 342.37 ± 28.42 | 327.75 ± 28.07 |
| External US marker | Small | 174.80 ± 26.47 | 160.36 ± 24.72 |
| | Medium | 301.38 ± 28.54 | 277.48 ± 19.84 |
| | Big | 374.92 ± 20.39 | 388.70 ± 20.33 |

Figure 3 shows, on the left, area traces from the manual and GUI markers of three different sets (blue=small area; red=medium area; yellow=large area). Figure 3 in the centre, shows point cloud of manual vs. marker data points. On the right, Figure 3 shows histograms of the difference of each data point; mean and +- standard deviation are represented by above it. From top to bottom, Figure 3 shows Struts marker (top), Lumen marker (centre) and External US marker (bottom).

Assessing the error between the expected area from ground truth marking to the area returned by the following methods was found to be 8.13 ± 3.15 mm² for the Struts Marker, 10.59 ± 4.62 mm² for the Lumen Marker and 20.36 ± 17.95 mm² for the External US Marker (Table 4).

**Table 4. Accuracy measurements.**

| | Struts | Lumen | External US |
|---|---|---|---|
| Average mean error | 8.13 mm² | 10.59 mm² | 20.36 mm² |
| Averaged SD | 3.15 mm² | 4.62 mm² | 17.95 mm² |

The semi-automatic markers GUI have been a great development in terms of speed with respect to the manual marking and the users' feedback has been positive in all cases, improving and increasing the productivity time.

The validation results show that the GUI is reliable, and the measurements stays within ±10% of the area in all the algorithms, however the external US has more variability and wider range of error than the one extracted from IVUS.

### Longitudinal temporal monitoring of the IVC using external ultrasound

The present disclosure further provides longitudinal temporal monitoring of the IVC using external ultrasound to repeatedly/conveniently capture the same cross-sectional slice while person undergoes manoeuvres.

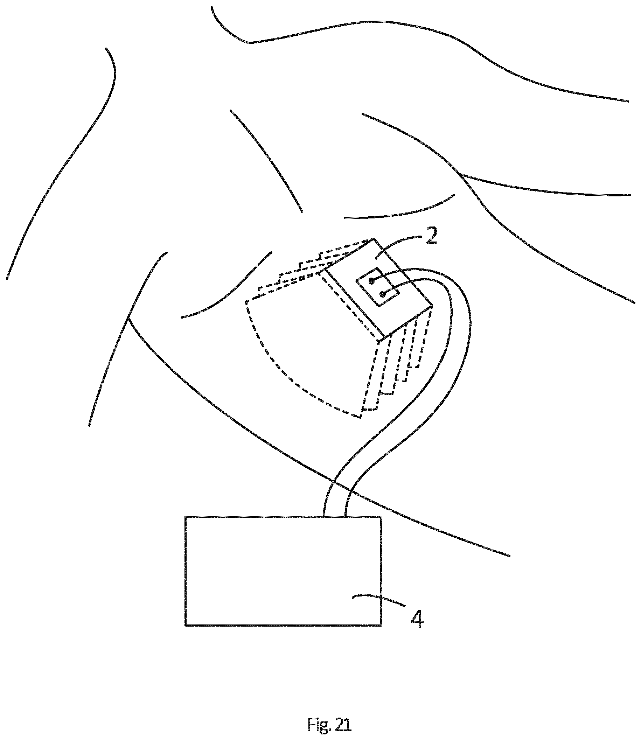

The images may be obtained from a body worn or internal ultrasound imaging system. A portable ultrasound device which may be used to obtain the ultrasound images according to one aspect of the present invention is shown in Figures 21 to 25. The portable ultrasound device is intended for use in the remote monitoring of heart failure (HF) patients, but it is not limited to this application as it is also suitable for use in other applications. With reference to Figure 1, the portable ultrasound device is shown in two parts, an ultrasound transducer 2 for securement to the torso and a linked console 4.

Ultrasound transducer 2 comprises a 2D array of independently controllable ultrasound transducer elements 6 for producing an ultrasonic pulse. Ultrasound transducer 2 is capable of 2D and 3D B-mode scanning in addition to functional Doppler modes such as Pulsed Wave, Colour and Power Doppler. The external 2D array of ultrasound transducer elements enables 3D imaging of the entire abdominal cavity, and assessment of both the anatomical structure and blood volumetric flowrates of the arterial and venous vessels, which would ideally be measured in the home by the patient, for long term monitoring by a clinical team.

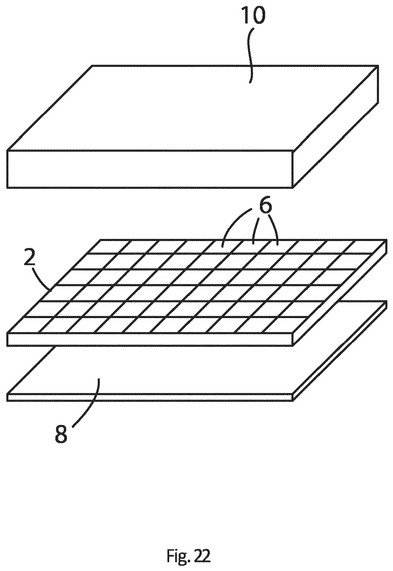

In the embodiment shown in Figure 22, transducer 2 comprises an array of N x M independently controlled ultrasonic elements 6, either in piezoelectric crystal or CMUT form, capable of beam steering throughout the thorax and abdomen.

The ultrasound transducer 2 in this embodiment extends to approximately 10cm in length, and approximately 5cm in width with 128 x 64 piezo crystals/CMUT cells. Depending on patient size the dimensions of the ultrasound transducer and the number of cells could vary (10cm x 5cm, 12cm x 6cm, 14cm x 7cm... etc.). The transmission frequency range of the ultrasound transducer varies between 2 - 8MHz, and this could be tuned in the initial training phase, dependent on the size and shape of the patient. Prior art ultrasound transducers/probes typically have an NxN square aperture. In the present embodiment, a rectangular transducer aperture (longer than the conventional smaller footprint transducers) allows improved extended-angle imaging capability of structures/organs of interest related to cardiac output and congestion.

It is intended that the patient would periodically affix the ultrasound transducer 2 to the body throughout a monitoring period of about one week. The ultrasound transducer 2 in this embodiment is secured to the torso by a pad 8 formed from an adhesive/gel/sponge like substance. The pad 8 provides an ultrasound transfer medium between the transducer and the skin.

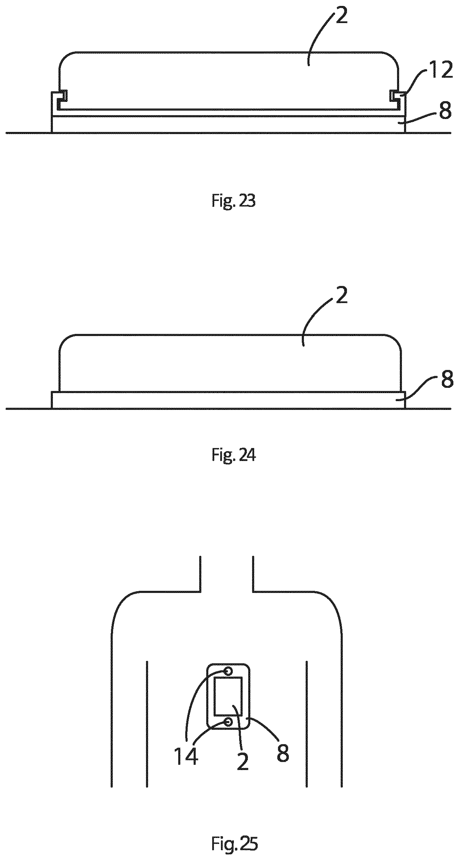

Figure 23 shows one embodiment of pad 8 that allows for daily reuse of the ultrasound transducer. By reusable, it is meant that the pad 8 can remain on the skin for the entire monitoring period. The pad 8 is provided with a mechanical locking plate 12 adapted to removably receive ultrasound transducer 2. In this embodiment the ultrasound transducer 2 slides within the locking plate 12 to secure it to the underlying adhesive pad and hence the torso, ready for use. Between readings, the ultrasound transducer can be removed from the patient by sliding it out from the locking plate. The adhesive pad incorporating the locking plate may remain on the skin until the next use without causing discomfort to the patient.

An alternative embodiment of pad in shown in Figure 24. In this embodiment a disposable gel or foam adhesive pad is provided, which is disposed of after each use. The pad has no locking plate, instead both the upper and lower surface of the pad are adhesive to attach on one side to the transducer and on the other to the skin. The pad is for one time use only and is completely removed from the skin when the transducer is not in use. To ensure that the transducer is periodically replaced in the same location on the torso throughout the monitoring period, the adhesive pad is provided with a plurality of guide holes 14 for alignment with corresponding markers on the skin.

Adhesion of the transducer 2 to the skin is not essential to the disclosure, therefore other forms of securement of the transducer to the torso are also envisaged.

As shown in figure 22, ultrasound transducer 2 further comprises a multiplexor 10. Ultrasound transducer 2 may further comprise a user interface (not shown) in order to start and stop/pause the examination procedure. The user interface may take the form of a button, a touch pad or other activation means. A signal generator (audio, visual or haptic buzzing etc.) may further be provided to prompt the patient to perform specific breathing manoeuvres to enhance the clinical signal, for example natural breathing (inhale/exhale), sudden sniff or Valsalva. An accelerometer may also be provided to enable the system to track the motion and position of the patient.

As shown in Figure 21, the ultrasound transducer is configured for application to the patient and connected to and controlled by control module 4. The control module or console 4 comprises a power source, processing means and a communications interface. The power source is a high voltage (HV) generator which applies an alternating potential difference across the piezo crystals/CMUT elements 6 in order to generate an ultrasonic pulse.

The processing means comprises a TX beam former for controlling the timing of each of the ultrasonic elements and to assist in the beam steering, in order that plane waves at angles to the transducer may be swept across the abdomen and anatomical structures of interest, as shown in figure 1. A further RX beamforming control system is provided within the processing means to receive and process the received ultrasonic signals and relay the processed received signals to an image processor where they are prepared for real-time grayscale display or analysis. In alternative embodiments a gain amplifier is used. The communications interface may be used for user interfacing, for display, for audio or for communication of results for remote analysis, for example to a remote clinical team.

The image processor may further be configured to compensate for changes in transducer position relative to the target abdominal structures, such that readings remain accurate regardless of transducer position.

The system of the present disclosure may further comprise a single element 1-D array transducer for imaging the lungs, examining for fluid (B-Lines) due to congestion. This may be used with, or form part of, the device of the present disclosure. The single element 1-D array transducer may be used to image B-lines. It may be connectable via a cable, to control module 4 or may be configured to communicate wirelessly with control module 4, for example using Bluetooth^{®}. Its use would be particularly advantageous if there was a risk of pulmonary congestion. The patient would manually apply the 1-D array ultrasound transducer to a number of sites (as per fig 25) on the chest for assessing congestion in the lungs. The patient would be prompted to position the ultrasound transducer in the specific areas; the patient would have received training initially and the display on the main module could be configured to prompt the patient on where to position the ultrasound transducer. In alternative embodiments, subcutaneous magnetic elements as described previously could be used.

The system may further comprise an auscultation device, in the form of a simple microphone receiver, and/or Forced Expiration Volume (FEV), embedded in a simple face mask, for connection to control module 4. This device would record sounds from the lungs over a respiratory cycle and also for forced expulsions/Valsalva in order to establish the extent of congestion (the extent of crackle/ wheezing of the lungs would be analysed). In addition, The FeV1/FEV ratios could be measured. These could be compared to a baseline set of results combining both the B-line analysis and the audio congestions/FEV analysis.

An ultrasound transducer such as that described above may be positioned and secured on the patient. To enable monitoring, the ultrasound transducer is placed on the same site of the patient daily or for a period of days. This could be assisted with the use of tattooing of the skin for repeatable placement of the transducer. Specific placement of the transducer could be decided upon on a patient-by-patient basis, depending on their anatomical profile and determined during an initial training/educational phase (for example in the hospital in consultation with healthcare professional). For remote monitoring, measurements are taken at the same time each day, with the patient in the same position, to ensure repeatability.

A device may be provided that auto locates the same IVC or SVC segment over time.

A mechanical or similar set-up that ensures that the device is over same approx. area (for example, this might be a belt with a dedicated holder & straps to ensure repeatable positioning).

Alternatively, or additionally, inked (permanent or the likes of semi-permanent) markers on the patient's body could be used for positioning. The device could recognise it's in the correct location using optical recognition of say 3 tattooed dots on the patient's body. This may be assisted by the device comprising a handheld probe shaped like a computer mouse.

A plurality of tattooed dots (or other shapes - such as asymmetric triangles such that both location and orientation can be precisely tracked) - either permanently or temporary could be used. These might be stencilled on in a doctor's office or be applied via ink on an adhesive positioned by a trained operator. Orientation is key to maximise possibly of achieving a consistent biplanar slice through the IVC.

In use, the holder for that day's reading may be lined up with those dots and then affixed using some type of adhesive. Any holder set-up straps etc. could be released and the individual let breathe freely during the reading.

The above may be coupled with feature extraction (e.g., liver, kidney, diaphragm, vessel branch or atrial structure recognition or other anatomical recognition) to identify the precise CSA required. This could be used with a 3D section or a 2D section.

Such a device would be able to locate this same IVC CSA/3D volume consistently over extended durations - months to years - and capture during relative motion of respiration cycles (combination of device itself moving with respiration and image analysis).

Such a device could trigger a series of prompts for patient to engage in appropriate manoeuvres (either a set sequence and/ or selected using some decision-making criteria). One example is as follows:
1) Today's reading is 300mm^2 IVC max + collapse of 50mm^2.
2) Yesterday's was 300mm^2 + 100mm^2.
3) Algorithm detects reduced collapse at same area (which we would believe is a sign of volume overload but could be hypo (depending on what area is 'normal')).
4) Algorithm then figures out that the right manoeuvre to test this is a sniff - so system instructs person to take a reading with a sniff in the middle to determine the optimal sequence to personalise the person on his/ her own P-V curve - continuously gather/ acquire images during the manoeuvre/ activity (would be fascinating for HFpEF patients in particular) - quasi stress-test (e.g., insight into splanchnic capacitance).
5) Device generates an IVC score.
6) IVC score used to provide therapeutic input.

An extended duration patch may be provided for continuous monitoring. It may have a rechargeable battery and/or may fit into a holder.

The words "comprises/comprising" and the words "having/including" when used herein with reference to the present disclosure are used to specify the presence of stated features, integers, steps, or components but do not preclude the presence or addition of one or more other features, integers, steps, components, or groups thereof.

It is appreciated that certain features of the disclosure, which are, for clarity, described in the context of separate embodiments, may also be provided in combination in a single embodiment. Conversely, various features of the disclosure which are, for brevity, described in the context of a single embodiment, may also be provided separately or in any suitable sub-combination.

## Claims

1. A computer-implemented method of determining measurements of the Inferior Vena Cava, IVC, using ultrasound imaging, comprising:
providing a three-dimensional, 3D, ultrasound image of a portion of the body in which the IVC is located by combining a plurality of 2-dimensional, 2D, ultrasound images into a three-dimensional, 3D, ultrasound stack, wherein the plurality of 2D ultrasound images are obtained by a body-worn ultrasound transducer array;
performing image analysis on the 3D ultrasound image to identify the IVC relative to other anatomical structures by identifying at least one anatomical structure in the 3D ultrasound image selected from the group containing the aorta, the renal arteries, the hepatic vein, the right atrium or the diaphragm, and identifying the IVC in the 3D ultrasound image by its known position relative to the identified anatomical structure;
selecting a single slice of the 3D ultrasound image, the slice comprising a cross-sectional image of the identified IVC; and
determining the cross-sectional area of the IVC from the cross-sectional image of the IVC in the selected slice.

2. The method of claim 1, wherein the plurality of 2D ultrasound images are obtained by the body-worn ultrasound transducer array from a plurality of directions or locations on the body.

3. The method of claim 1, wherein the plurality of 2D ultrasound images are obtained using beam forming.

4. The method of claim 1, wherein identifying at least one anatomical structure in the image comprises the use of Doppler data to identify the direction and velocity of blood flow.

5. The method of claim 1 or claim 4, wherein identifying at least one anatomical structure in the image comprises the use of known geometric properties of the at least one anatomic structure.

6. The method of claim 1, wherein performing image analysis on the 3D ultrasound image to identify the IVC relative to other anatomical structures comprises identifying anatomical structures using edge detection techniques.

7. The method of claim 1, further comprising verifying the identification of the IVC in the image using an additional metric selected from a group comprising Doppler velocity, direction of blood flow, pulsatility, distances, pulmonary B-line measurements and audio respiratory data.

8. The method of any preceding claim, wherein determining the cross-sectional area of the IVC from the cross-sectional image of the IVC in the selected slice comprises
using edge detection techniques to identify the wall of the IVC; or
the fitting of an ellipse to the IVC; or
the use of thresholding techniques.

9. The method of any preceding claim, wherein the steps of providing, performing, selecting and determining are repeated over time to obtain a series of measurements.

10. The method of any preceding claim wherein determining the cross-sectional area of the IVC from the cross-sectional image of the IVC in the selected slice comprises the use of a neural network to mark the IVC in the ultrasound image.

11. A computer program product comprising instructions which, when the program is executed by a computer, cause the computer to carry out the method of any of any preceding claim.

12. A computer-readable storage medium comprising instructions which, when executed by a computer, cause the computer to carry out the method of any of claims 1 to 10.

## Patentansprüche

1. Ein computerimplementiertes Verfahren zum Bestimmen von Messungen der unteren Hohlvene, IVC (IVC = Inferior Vena Cava), mittels Ultraschallbildgebung, das Folgendes aufweist:
Bereitstellen eines dreidimensionalen, 3D, Ultraschallbilds eines Körperbereichs, in dem sich die IVC befindet, durch Zusammenfügen einer Vielzahl von zweidimensionalen, 2D, Ultraschallbildern zu einem dreidimensionalen, 3D, Ultraschall-Stack, wobei die Vielzahl von 2D-Ultraschallbildern durch ein am Körper getragenes Ultraschallwandler-Array erhalten wird;
Durchführen einer Bildanalyse an dem 3D-Ultraschallbild zum Identifizieren der IVC relativ zu anderen anatomischen Strukturen durch Identifizieren wenigstens einer anatomischen Struktur in dem 3D-Ultraschallbild, ausgewählt aus der Gruppe, die die Aorta, die Nierenarterien, die Lebervene, das rechte Atrium oder das Zwerchfell enthält, und Identifizieren der IVC in dem 3D-Ultraschallbild anhand ihrer bekannten Position relativ zu der identifizierten anatomischen Struktur;
Auswählen einer einzigen Schicht des 3D-Ultraschallbilds, wobei die Schicht ein Querschnittsbild der identifizierten IVC aufweist; und
Bestimmen der Querschnittsfläche der IVC aus dem Querschnittsbild der IVC in der ausgewählten Schicht.

2. Das Verfahren nach Anspruch 1, wobei die Vielzahl von 2D-Ultraschallbildern durch das am Körper getragene Ultraschallwandler-Array aus einer Vielzahl von Richtungen oder Positionen am Körper erhalten wird.

3. Das Verfahren nach Anspruch 1, wobei die Vielzahl von 2D-Ultraschallbildern mittels Beamforming erhalten wird.

4. Das Verfahren nach Anspruch 1, wobei das Identifizieren wenigstens einer anatomischen Struktur in dem Bild die Verwendung von Doppler-Daten zum Identifizieren der Richtung und Geschwindigkeit des Blutflusses aufweist.

5. Das Verfahren nach Anspruch 1 oder Anspruch 4, wobei das Identifizieren wenigstens einer anatomischen Struktur in dem Bild die Verwendung bekannter geometrischer Eigenschaften der wenigstens einen anatomischen Struktur aufweist.

6. Das Verfahren nach Anspruch 1, wobei das Durchführen einer Bildanalyse an dem 3D-Ultraschallbild zum Identifizieren der IVC relativ zu anderen anatomischen Strukturen das Identifizieren anatomischer Strukturen mittels Kantendetektionstechniken aufweist.

7. Das Verfahren nach Anspruch 1, das ferner das Verifizieren der Identifizierung der IVC in dem Bild mittels einer zusätzlichen Metrik aufweist, ausgewählt aus einer Gruppe, die Doppler-Geschwindigkeit, Richtung des Blutflusses, Pulsatilität, Abstände, pulmonale B-Linien-Messungen und Audio-Atemdaten aufweist.

8. Das Verfahren nach einem vorhergehenden Anspruch, wobei das Bestimmen der Querschnittsfläche der IVC aus dem Querschnittsbild der IVC in der ausgewählten Schicht
das Verwenden von Kantendetektionstechniken zum Identifizieren der Wand der IVC; oder
das Anpassen einer Ellipse an die IVC; oder
das Verwenden von Schwellenwertverfahren aufweist.

9. Das Verfahren nach einem vorhergehenden Anspruch, wobei die Schritte des Bereitstellens, Durchführens, Auswählens und Bestimmens über die Zeit wiederholt werden, um eine Reihe von Messungen zu erhalten.

10. Das Verfahren nach einem vorhergehenden Anspruch, wobei das Bestimmen der Querschnittsfläche der IVC aus dem Querschnittsbild der IVC in der ausgewählten Schicht die Verwendung eines neuronalen Netzes zum Markieren der IVC in dem Ultraschallbild aufweist.

11. Ein Computerprogrammprodukt, das Anweisungen aufweist, die, wenn das Programm durch einen Computer ausgeführt wird, den Computer veranlassen, das Verfahren nach einem der vorhergehenden Ansprüche durchzuführen.

12. Ein computerlesbares Speichermedium, das Anweisungen aufweist, die, wenn sie durch einen Computer ausgeführt werden, den Computer veranlassen, das Verfahren nach einem der Ansprüche 1 bis 10 durchzuführen.

## Revendications

1. Procédé mis en œuvre par ordinateur de détermination de mesures de la veine cave inférieure, VCI, au moyen d'une imagerie ultrasonore, comprenant :
la fourniture d'une image ultrasonore tridimensionnelle, 3D, d'une partie du corps dans laquelle est située la VCI, par combinaison d'une pluralité d'images ultrasonores bidimensionnelles, 2D, en un empilement ultrasonore tridimensionnel, 3D, dans lequel la pluralité d'images ultrasonores 2D sont obtenues par un réseau de transducteurs ultrasonores porté sur le corps ;
la réalisation d'une analyse d'image sur l'image ultrasonore 3D pour identifier la VCI par rapport à d'autres structures anatomiques, par identification, dans l'image ultrasonore 3D, d'au moins une structure anatomique choisie dans le groupe contenant l'aorte, les artères rénales, la veine hépatique, l'oreillette droite ou le diaphragme, et identification de la VCI dans l'image ultrasonore 3D par sa position connue par rapport à la structure anatomique identifiée ;
la sélection d'une coupe unique de l'image ultrasonore 3D, la coupe comprenant une image en coupe transversale de la VCI identifiée ; et
la détermination de l'aire de section transversale de la VCI à partir de l'image en coupe transversale de la VCI dans la coupe sélectionnée.

2. Procédé selon la revendication 1, dans lequel la pluralité d'images ultrasonores 2D sont obtenues par le réseau de transducteurs ultrasonores porté sur le corps à partir d'une pluralité de directions ou d'emplacements sur le corps.

3. Procédé selon la revendication 1, dans lequel la pluralité d'images ultrasonores 2D sont obtenues au moyen d'une formation de faisceau.

4. Procédé selon la revendication 1, dans lequel l'identification d'au moins une structure anatomique dans l'image comprend l'utilisation de données Doppler pour identifier la direction et la vitesse du flux sanguin.

5. Procédé selon la revendication 1 ou la revendication 4, dans lequel l'identification d'au moins une structure anatomique dans l'image comprend l'utilisation de propriétés géométriques connues de l'au moins une structure anatomique.

6. Procédé selon la revendication 1, dans lequel la réalisation d'une analyse d'image sur l'image ultrasonore 3D pour identifier la VCI par rapport à d'autres structures anatomiques comprend l'identification de structures anatomiques au moyen de techniques de détection de contours.

7. Procédé selon la revendication 1, comprenant en outre la vérification de l'identification de la VCI dans l'image au moyen d'une métrique supplémentaire choisie dans un groupe comprenant la vitesse Doppler, la direction du flux sanguin, la pulsatilité, des distances, des mesures de lignes B pulmonaires et des données respiratoires audio.

8. Procédé selon l'une quelconque des revendications précédentes, dans lequel la détermination de l'aire de section transversale de la VCI à partir de l'image en coupe transversale de la VCI dans la coupe sélectionnée comprend :
l'utilisation de techniques de détection de contours pour identifier la paroi de la VCI ; ou
l'ajustement d'une ellipse à la VCI ; ou
l'utilisation de techniques de seuillage.

9. Procédé selon l'une quelconque des revendications précédentes, dans lequel les étapes de fourniture, de réalisation, de sélection et de détermination sont répétées au cours du temps pour obtenir une série de mesures.

10. Procédé selon l'une quelconque des revendications précédentes, dans lequel la détermination de l'aire de section transversale de la VCI à partir de l'image en coupe transversale de la VCI dans la coupe sélectionnée comprend l'utilisation d'un réseau de neurones pour marquer la VCI dans l'image ultrasonore.

11. Produit programme d'ordinateur comprenant des instructions qui, lorsque le programme est exécuté par un ordinateur, amènent l'ordinateur à mettre en œuvre le procédé selon l'une quelconque des revendications précédentes.

12. Support de stockage lisible par ordinateur comprenant des instructions qui, lorsqu'elles sont exécutées par un ordinateur, amènent l'ordinateur à mettre en œuvre le procédé selon l'une quelconque des revendications 1 à 10.
